**Europäisches Patentamt**

**European Patent Office**

**Office européen des brevets**

(19)

(11) Veröffentlichungsnummer: **0 095 142**
**B2**

(12)                    **NEUE EUROPÄISCHE PATENTSCHRIFT**

(45) Veröffentlichungstag der neuen Patentschrift:
**12.10.88**

(51) Int. Cl.⁴: **A 61 H 21/00, A 61 M 31/00**

(21) Anmeldenummer: **83104937.4**

(22) Anmeldetag: **19.05.83**

(54) **Hämorrhoidenbehandlungsstab.**

(30) Priorität: **26.05.82 DE 3219727**

(43) Veröffentlichungstag der Anmeldung:
**30.11.83 Patentblatt 83/48**

(45) Bekanntmachung des Hinweises auf die Patenterteilung:
**16.04.86 Patentblatt 86/16**

(45) Bekanntmachung des Hinweises auf die Entscheidung
über den Einspruch:
**12.10.88 Patentblatt 88/41**

(84) Benannte Vertragsstaaten:
**AT BE CH DE FR GB IT LI LU NL SE**

(56) Entgegenhaltungen:
**FR-A-25 058**
**FR-A-2 192 534**
**GB-A-253 006**

(73) Patentinhaber: **Brühl, Wilhelm, Dr., Röntorfer
Strasse 3, D-4973 Vlotho Steinbründorf (DE)**
Patentinhaber: **Gebhardt, Bernd, Dipl.- Ing.,
Memelstrasse 19, D-4900 Herford (DE)**

(72) Erfinder: **Brühl, Wilhelm, Dr., Röntorfer Strasse 3,
D-4973 Vlotho Steinbründorf (DE)**
Erfinder: **Gebhardt, Bernd, Dipl.- Ing.,
Memelstrasse 19, D-4900 Herford (DE)**

(74) Vertreter: **Loesenbeck, Karl- Otto, Dipl.- Ing.,
Jöllenbecker Strasse 164, D-4800 Bielefeld 1 (DE)**

EP 0 095 142 B2

## Beschreibung

Die Erfindung betrifft einen Hämorrhoidenbehandlungsstab in Form eines analdehnenden Kegels, auf dessen Außenmantel seitliche Austrittsöffnungen münden, die von einem Zuführungskanal ausgehen.

Die Hämorrhoidenbeschwerden haben ihren Ursprung überwiegend in feinsten, längs verlaufenden Rissen im Afterkanal sowie auch in schwalbennesterartigen, nach oben geöffneten Taschen im Afterkanal, den sogenannten Krypten. In geringem Umfang sind auch quer verlaufende Risse vorzufinden. Bei der Stuhlentleerung dehnt sich der Afterschließmuskel. Damit dehnen sich auch die längs verlaufenden Risse, so daß sich der Stuhl darin festsetzen kann. Insbesondere weicher Stuhl kann bei der Passage auch in die Krypten und die quer verlaufenden Risse gedrückt werden. An sich wirkungsvolle Hämorrhoidensalben lassen eine wirklich erfolgreiche Behandlung nur dann erwarten, wenn es gelingt, diese Salbe auch tatsächlich in die Risse und gegebenenfalls Krypten hineinzupraktizieren. Bekannte Hämorrhoidenbehandlungsstäbe der gattungsgemäßen Art (FR-A-2 192 534) ermöglichen zwar aufgrund ihrer von der Spitze des Behandlungsstabes ausgehenden kegelförmigen Aufweitung eine Spreizung des Afterkanales und damit auch eine Spreizung der längs verlaufenden Risse, sie haben jedoch einen vollkommen gleichförmigen Kegelaußenmantel, auf dem die Austrittsöffnungen relativ scharfkantig münden. Aufgrund dieser Tatsache kann vielfach die Salbe nicht tief genug in die gespreizten Risse hineinpraktiziert werden. Auch kann das relativ scharfkantige Münden der Austrittsöffnungen außen auf dem Kegelaußenmantel bei der außerordentlichen Empfindlichkeit der Analschleimhäute Schmerzen hervorrufen. Ein sinnvolles Hineindrücken von Salbe in die taschenförmigen Krypten und etwaige quer verlaufende Risse ist nicht möglich.

Es ist ferner ein Hämorrhoidenbehandlungsstab bekannt (FR-A-25 058), dessen verdickte Spitze in Form eines kurzen analdehnenden Kegelabschnittes ausgebildet ist, an den sich dann nach hinten ein demgegenüber dünnerer Abschnitt anschließt, der in Kegellängsachsenrichtung orientierte scharfkantig auf der Mantelfläche mündende Nuten oder Rillen hat, wobei im Nut- bzw. Rillengrund seitliche Austrittsöffnungen münden, die von einem Zuführungskanal ausgehen. Soweit der angesprochene Abschnitt mit den Nuten bzw. Rillen Kegelform hat, verläuft die Kegelform einer Analdehnung genau entgegengesetzt. Eine Behandlung der längs verlaufenden Risse ist nicht sinnvoll möglich, da dort, wo die Salbe aus den Nuten bzw. Rillen austritt, keine Analdehnung stattfindet. Es tritt sogar der gegenteilige Effekt dadurch ein, daß sich die wulstige Wand des Analkanales, ohne gespreizt zu werden, in die Nuten bzw. Rillen hineindrückt. Mit dem dort gezeigten einen großen Übergangsradius aufweisenden flachen Übergangsbereich zwischen der verdickten Kegelspitze und dem die Nuten aufweisenden Abschnitt wird nicht das Einschieben von Salbe in die taschenförmigen Krypten oder Querrisse ermöglicht, und zwar umso weniger, als die unmittelbar angrenzende Analdehnungszone durch die Kegelspitze die Krypten und Querrisse eher zudrückt. Ein Hineinpraktizieren in nach unten zur Analöffnung hin gerichtete Krypten ist ausgeschlossen.

Der vorliegenden Erfindung liegt die Aufgabe zugrunde, einen Hämorrhoidenbehandlungsstab der gattungsgemäßen Art zu schaffen, der ein tiefes Hineinpraktizieren der Salbe in die Risse und die Krypten gewährleistet.

Die erfindungsgemäße Lösung besteht darin, daß der Kegel als Außenmantelkontur eine gleichförmig über den Umfang umlaufende Wellenkontur hat, deren Berge und Täler in Kegellängsachsenrichtung verlaufen, wobei die Austrittsöffnungen jeweils in den Tälern münden.

Dank dieser Ausgestaltung werden die längs verlaufenden Risse im Analkanal nicht nur ausreichend gespreizt, dank der Wellenkontur des Außenmantels des Kegels bleibt die Spreizung auch aufrechterhalten und durch Drehen des Behandlungsstabes kann dank der Wellenkontur die Salbe auch tief in die Risse hineinpraktiziert werden. Dabei besteht eine Gefahr einer vorzeitigen Salbenabstreifung nicht, da durch die Mündung der Austrittsöffnungen in den Tälern immer ausreichend Salbe zur Verfügung steht. Die Berge und Täler der Wellenkontur wirken einmassierend. Durch die ausreichende Zurverfügungstellung von Salbe und durch die spezielle Wellenkontur des Außenmantels, die das Verbleiben der Krypten und auch quer verlaufender Risse in einer die Aufnahme begünstigenden Stellung ermöglicht, kann auch durch mehrfaches Hin- und Herziehen des Kegels insoweit ein Einschieben der Salbe erfolgen. Dieser Effekt kann noch dadurch erheblich unterstützt werden, daß gemäß einer bevorzugten Ausführungsform des Hämorrhoidenbehandlungsstabes in der Nähe der Kegelspitze von dem Außenmantel ein gerundeter, umlaufender Wulst vorsteht.

Der Wulst befindet sich somit in einem Bereich, der praktisch keine Analdehnung mehr hervorruft, so daß es insoweit auch nicht quasi zu einem Zuziehen von quer verlaufenden Rissen oder Kryptenöffnungen kommen kann. Der über den Mantel vorspringende Wulst ermöglicht dann bei der Schiebebewegung des Behandlungsstabes auch das Einpraktizieren der Salbe, insbesondere auch in solche Krypten, deren Öffnungen nach hinten und vorne weisen.

Weitere bevorzugte Ausgestaltungen eines derartigen Hämorrhoidenbehandlungsstabes sind in weiteren abhängigen Ansprüchen gekennzeichnet. Ein Ausführungsbeispiel eines derartigen Hämorrhoidenbehandlungsstabes wird nachstehend unter Bezugnahme auf die Zeichnung näher beschrieben.

Es zeigen

Figur 1 einen Hämorrhoidenbehandlingsstab gemäß der Erfindung im Längsschnitt,

Figur 2 einen Querschnitt gemäß Schnitt II' II der Figur 1.

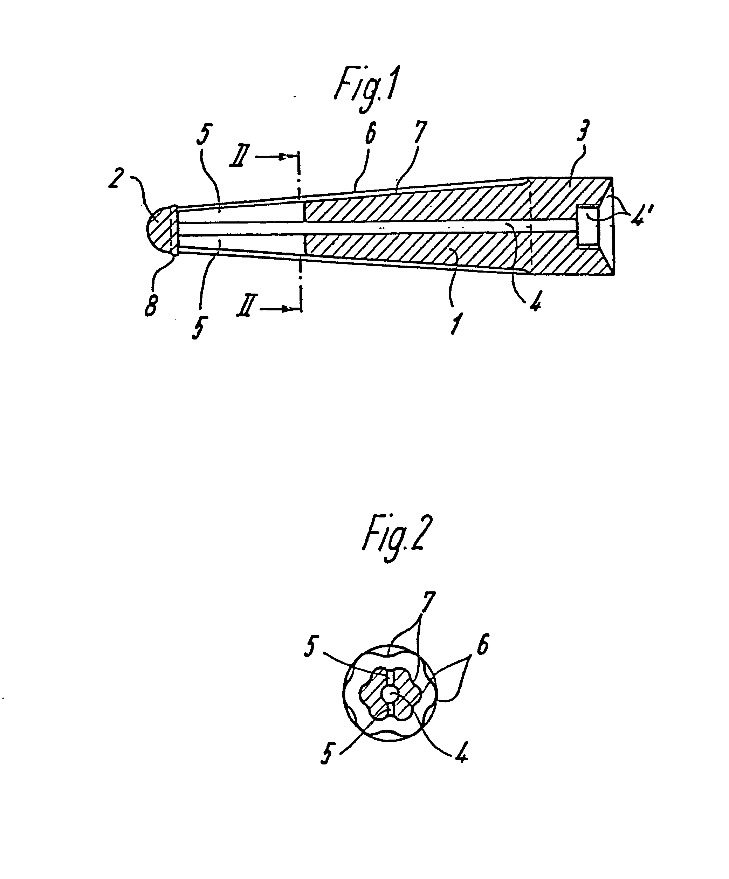

Der dargestellte Hämorrhoidenbehandlungsstab ist in seiner Grundform kegelförmig. Der den Hauptkörper bildende Kegel 1 hat eine gerundete Kegelspitze 2. An der Basis befindet sich ein zylindrischer Absatz 3, der einen Schraubanschluß 4' für den Schraubstutzen einer Salbentube hat, dergestalt, daß der Behandlungsstab als solcher auf die Salbentube geschraubt werden kann.

Mittig in dem Kegel 1 und dem zylindrischen Ansatz 3 ist ein Zuführungskanal 4 vorgesehen, der sich bis in die Nähe der Kegelspitze 2 erstreckt und von dem im Bereich der Kegelspitze seitliche Austrittsöffnungen 5 abzweigen, die im dargestellten Ausführungsbeispiel als zwei längliche, sich diametral gegenüberliegende Austrittsschlitze ausgebildet sind.

Die Außenmantelkontur des Kegels 1 weist Erhöhungen und Vertiefungen auf. Im dargestellten Ausführungsbeispiel hat die Außenmantelkontur hierzu gleichförmig über den Umfang verteilte, sich von dem zylindrischen Ansatz 3 bis nahe an die Kegelspitze 2 erstreckende Wellen, deren Berge 6 und deren Täler 7 in Kegellängsachsenrichtung orientiert sind. Die Mündungsöffnungen der seitlichen Austrittsschlitze 5 liegen dabei jeweils in einem Tal 7.

Zwischen der gerundeten Kegelspitze 2 und dem ihr zugewandt liegenden Endbereich der seitlichen Austrittsschlitze 5 ist auf den Außenmantel des Kegels 1 ein umlaufender Wulst 8 geformt, der ein wenig über die Außenmantelkontur, also auch noch über die Wellenberge 6, vorsteht. Aufgrund dieser Tatsache hilft er bei einer Rückzichbewegung des Hämorrhoidenbehandlungsstabes mit, Salbe in die taschenförmigen Krypten zu drücken.

Der mit der Salbentube verschraubte Behandlungsstab wird in den After eingeführt. Der Kegel 1 ist so bemessen, daß er als Analdehner wirkt. Bei hierzu zweckentsprechendem Basisdurchmesser hat sich in diesem Zusammenhang eine Kegellänge von ca. 8 - 10 cm bei einem Kegelwinkel von ca. 10° als zweckmäßig erwiesen.

Nach der Analdehnung, die mit einer Dehnung der haarfeinen Risse verbunden ist, wird mittels der angeschraubten Tube Salbe durch den Zuführungskanal 4 und aus den seitlichen Austrittsschlitzen 5 heraus in den Afterkanal gedrück. Durch ein Hin- und Herdrehen des Behandlungsstabes wird die Salbe, gerade auch durch die wellenförmige Konturierung des Außenmantels des Kegels 1, in die aufgedehnten Risse tief hineinmassiert. Durch ein mehrfaches Zurückschieben und Wiedervorziehen des Behandlungsstabes können auch, unterstützt durch den vorstehenden Wulst 8, die taschenförmigen Krypten mit Salbe gefüllt werden.

## Patentansprüche

1. Hämorrhoidenbehandlungsstab in Form eines analdehnenden Kegels (1), auf dessen Außenmantel seitliche Austrittsöffnungen (5) münden, die von einem Zuführungskanal (4) ausgehen, <u>dadurch gekennzeichnet,</u> daß der Kegel (1) als Außenmantelkontur eine gleichförmig über seinen Umfang verlaufende Wellenkontur hat, deren Berge (6) und Täler (7) in Kegellängsachsenrichtung verlaufen, wobei die Austrittsöffnungen (5) jeweils in den Tälern (7) münden.

2. Hämorrhoidenbehandlungsstab nach Anspruch 1, dadurch gekennzeichnet, daß in der Nähe der Kegelspitze (2) von dem Außenmantel des Kegels (1) ein gerundeter, umlaufender Wulst (8) vorsteht.

3. Hämorrhoidenbehandlungsstab nach Anspruch 1, dadurch gekennzeichnet, daß die seitlichen Austrittsöffnungen durch zwei einander diametral gegenüberliegende längliche Austrittsschlitze (5) gebildet sind.

4. Hämorrhoidenbehandlungsstab nach Anspruch 2, dadurch gekennzeichnet, daß der Wulst (8) zwischen der gerundeten Kegelspitze (2) und den Austrittsöffnungen (5) angeordnet ist.

## Claims

1. A haemorrhoidal treatment plug in the form of an anus-dilating cone (1) with a jacket in which lateral outlets (5), branching off from an inlet duct (4), are ending, characterized in that the jacket of said cone (1) has a wavy contour evenly spread over its periphery with peaks (6) and valleys (7) extending in the direction of the longitudinal cone axis, and with each of said outlets (5) terminating in one of said valleys (7).

2. A haemorrhoidal treatment plug as described in Claim 1 above, characterized in that a rounded peripheral ridge (8) projects from the jacket of said cone (1) adjacent to the cone tip (2).

3. A haemorrhoidal treatment plug as described in Claim 1 above, characterized in that said lateral outlets are formed of two elongated outlet slots (5) in diametrically opposite positions.

4. A haemorrhoidal treatment plug as described in Claim 2 above, characterized in that said ridge (8) is arranged between said rounded cone tip (2) and said outlets (5).

**Revendications**

1. Bâtonnet de traitement des hémorroïdes sous forme d'un cône (1), écarteur anal, sur la paroi externe duquel des ouvertures latérales de décharge (5) débouchent, celles-ci partant d'un canal d'amenée (4), caractérisé par le fait que le contour de la paroi extérieure du cône (1) a un contour ondulé réparti uniformèment sur la pèriphérie dont les protubérances (6) et les creux (7) vont dans 1e sens de l'axe longitudinal du cône, les ouvertures de décharge (5) débouchant a chaque fois dans les creux (7).

2. Bâtonnet de traitement des hémorroïdes selon la revendication 1, caractérisé par le fait qu'à proximité du sommet du cône (2) il y a un bourrelet (8) continu, arrondi qui fait seillie tout autour à partir de la paroi extérieure.

3. Bâtonnet de traitement des hémorroïdes selon la revendication 1, caractérisé par le fait que les ouvertures latérales de décharge sont formées par deux fentes longitudinales d'émission (5) diamétralement opposées.

4. Bâtonnet de traitement des hémorroïdes selon la revendication 2, caractérisé par le fait que le bourrelet (8) est disposé entre le sommet arrondi (2) du cône et les ouvertures latérales de décharge (5).

Fig.1

Fig.2